(19) Europäisches Patentamt
European Patent Office
Office européen des brevets

(11) Publication number: **0 405 516 A2**

(12) **EUROPEAN PATENT APPLICATION**

(21) Application number: 90112281.2

(22) Date of filing: 27.06.90

(51) Int. Cl.5: C07C 69/30, C09K 19/14, C07C 39/16

(30) Priority: 28.06.89 IT 2101789

(43) Date of publication of application: 02.01.91 Bulletin 91/01

(84) Designated Contracting States: AT BE CH DE DK ES FR GB LI NL SE

(71) Applicant: TECNOPART S.r.l.
16, Piazza della Repubblica
Milan(IT)

(72) Inventor: Dalcanale, Enrico, Dr.
26, Via Turbigo
I-28067 Pernate, Novara(IT)
Inventor: Bonsignore, Stefanio
8, Via Bergamo
I-28100 Novara(IT)
Inventor: Cometti, Giuseppe
14, Via Crocetta
I-28048 Verbania-Pallanza, Novara(IT)
Inventor: Du Vosel, Annick, Dr.
2, Cascina Molinaccio
I-28010 Sologno, Novara(IT)
Inventor: Soncini, Paolo, Dr.
26, Via G. Ferraris
I-28100 Novara(IT)
Inventor: Guglielmetti, Gianfranco
15/a, Via 4 Novembre
I-28010 Bogogno, Novara(IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

# (54) Ferroelectric, pyramidal liquid crystals.

(57) Described are dodeca-substituted derivatives of the macrocyclic tetramers obtainable by means of the acid-catalysed condensation of pyrogallol and formaldehyde, wherein the substituents are alkanoyl chains.

These substituted tetramers are optically anisotropic in the molten state and in particular show mesophases of pyramidal columnar type.

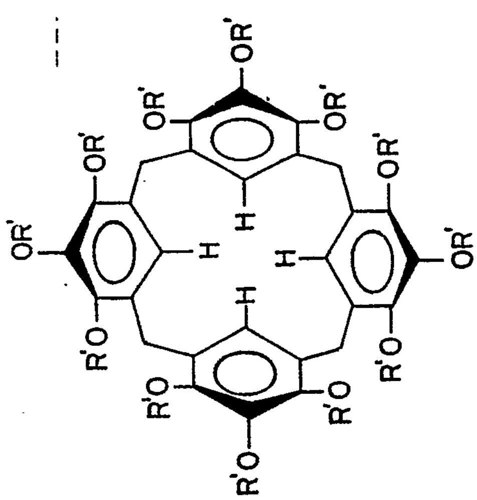

Fig.1

EP 0 405 516 A2

# FERROELECTRIC, PYRAMIDAL LIQUID CRYSTALS

The present invention relates to liquid-crystalline macrocyclic tetramers.

Particularly, the present invention relates to thermotropic dodeca-substituted macrocyclic tetramers, wherein the mesophases are of the pyramidal columnar type.

The term "pyramidal columnar mesophases", as used herein and in the appended claims, denotes a structure oriented along two dimensions, in which the molecules are arranged as columns. The molecules which the columns are composed of are formed by a central core of pyramidal shape, with the twelve chains of the substituents being symmetrically distributed around the base of the pyramid, as shown in Figure 1. In the column, the central cores are stacked on each other with the same orientation.

This type of mesophase is described, e.g., in "Journal de Physique" 47 (1986), page 351 and in "Zeitschrift für Naturforschung" 40A (1985), page 149.

An application example of columnar liquid crystals is given in US-A-4,430,650. Other examples of pyramidal liquid crystals are to be found in US-A-4,619,788 (wherein hexa-substituted derivatives of tribenzocyclononatriene are disclosed) and in IT-A-21,308 A/88 (disclosing dodeca-substituted derivatives of macrocyclic tetramers).

However, the products disclosed in the latter patent application, although displaying characteristics which are typical of thermotropic pyramidal liquid crystals, are not able to yield a hysteresis effect when subjected to inversion of polarization by means of an electrical field.

It has now been found that the above property is shown by certain dodeca-substituted derivatives of the cyclic tetramer obtainable from the acid-catalysed condensation of pyrogallol and form aldehyde, wherein the substituents are alkanoyl chains. These products are stable and anisotropic in the molten state, and show mesophases of the pyramidal type.

Therefore, the subject-matter of the instant invention are thermotropic, dodeca-substituted, macrocyclic tetramers of general formula I:

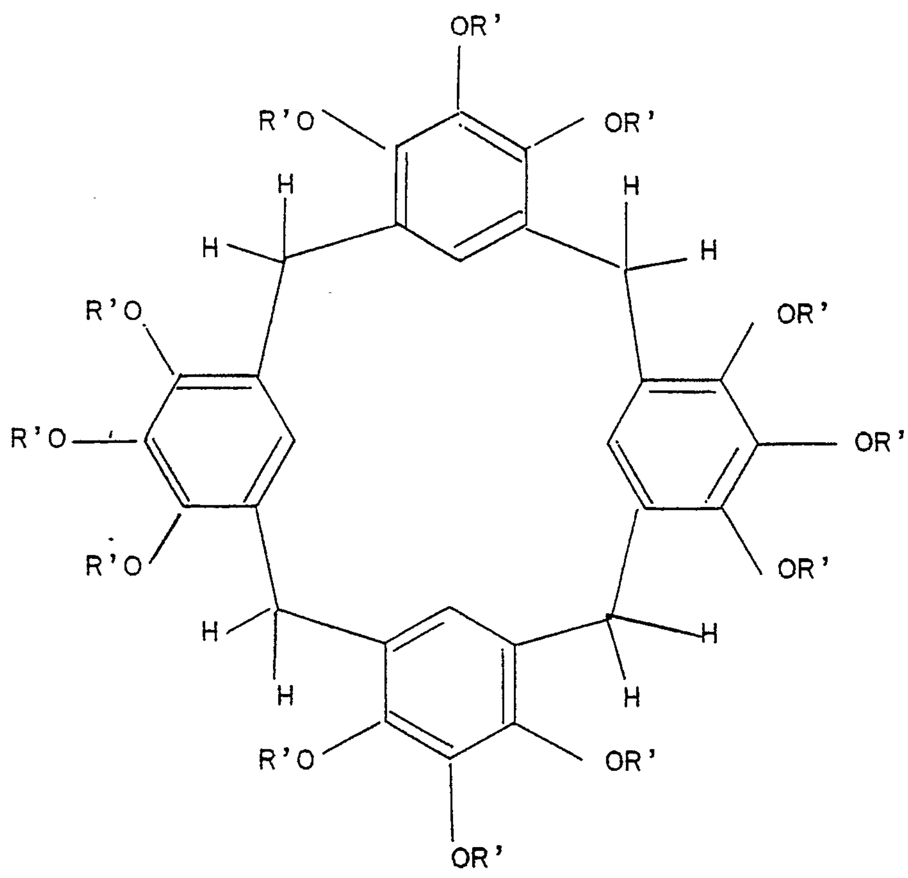

(I)

wherein the groups R′, identical or different from one another, represent a radical of general formula II:
-COR (II)
wherein R is a linear alkyl radical containing a number of carbon atoms larger than or equal to 8. Among the compounds of general formula I those in which R is a linear alkyl radical containing from 8 to 13 carbon atoms are preferred.

The substituents R′ of a molecule can be identical or different from one another. However, the compounds in which all radicals R′ are the same are preferred.

The compounds of general formula I preferably have clearing points of from about 50 to about 100 °C. Also preferred are those compounds whose temperatures of formation of the mesophase, starting from the isotropic molten phase, range from about 50 to about 70 °C and which show crystallization temperatures of form about 30 to about 50 °C.

The pyramidal, columnar organization of the liquid crystal phase can be evidenced by means of differential scanning calorimetry (DSC), analysis by optical microscope under polarized light and by X-ray diffraction. In particular, for all of the mesogens belonging to this family, a mesophase of the "fan shaped" type is observed, which is typical of highly organized columnar liquid crystals. By sliding the mesophase on a rigid support, a planar alignment of the molecules relative to the surface of the support (glass slide) can be observed.

The X-ray diffraction cf non-oriented mesophases shows, besides two very broad peaks (at 0.45 and 0.69 nm) typical of the molten state, also at least two sharp peaks (at 2.14 and 1.99 nm) which are indicative of the bidimensional organization of the columns.

In Figure 2 the transition temperatures between the various phases are reported, for exemplifying purposes, for some macrocyclic tetramers according to the present invention, wherein
$R' = -CO-C_nH_{2n-1}$.
In the figure, the temperature values are given on the ordinate and the values of n are shown on the abscissa; I, C and K indicate the isotropic phase, the pyramidal columnar liquid crystalline phase, and the crystalline phase, respectively, In particular, in the plot of Figure 2, the triangles represent the temperatures of formation of the mesophase, and the squares represent the columnar mesophase-crystal transitions and, in case a mesophase does not exist, the isotropic liquid-crystal transitions.

The thermotropic macrocyclic tetramers according to the present invention may be prepared by reacting a macrocyclic tetramer of general formula III:

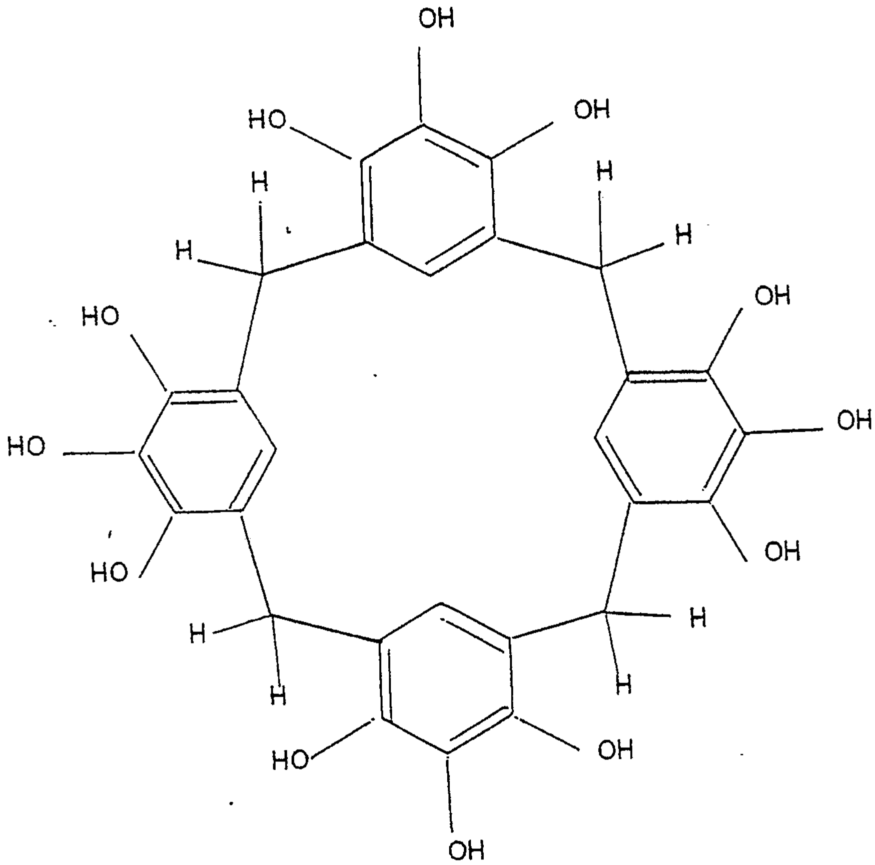

(III)

with acyl halides of general formula R′-X in which X represents a halogen atom (e.g. F, Cl, Br and I), preferably a chlorine atom, and R′ has the meaning given above. Examples of suitable acyl halides are: nonanoyl chloride, decanoyl chloride, undecanoyl chloride, dodecanoyl chloride, tetradecanoyl chloride, etc.

The reaction can be carried out as a normal esterification in bulk and at a temperature of from about 50 to about 200 °C.

The macrocyclic tetramer of formula III may be obtained by means of the acid-catalysed condensation of pyrogallol and paraformal dehyde at about 60 °C in ethanol, under anhydrous conditions. The catalyst can be selected, e.g., from hydrogen chloride, sulfuric acid, phosphoric acid, para-toluene-sulfonic acid, etc. Said catalyst generally is used in amounts of from 1 to 20% by weight, based on the total reactant mixture.

More particularly, the substrate of formula III may be prepared according to the following reaction scheme:

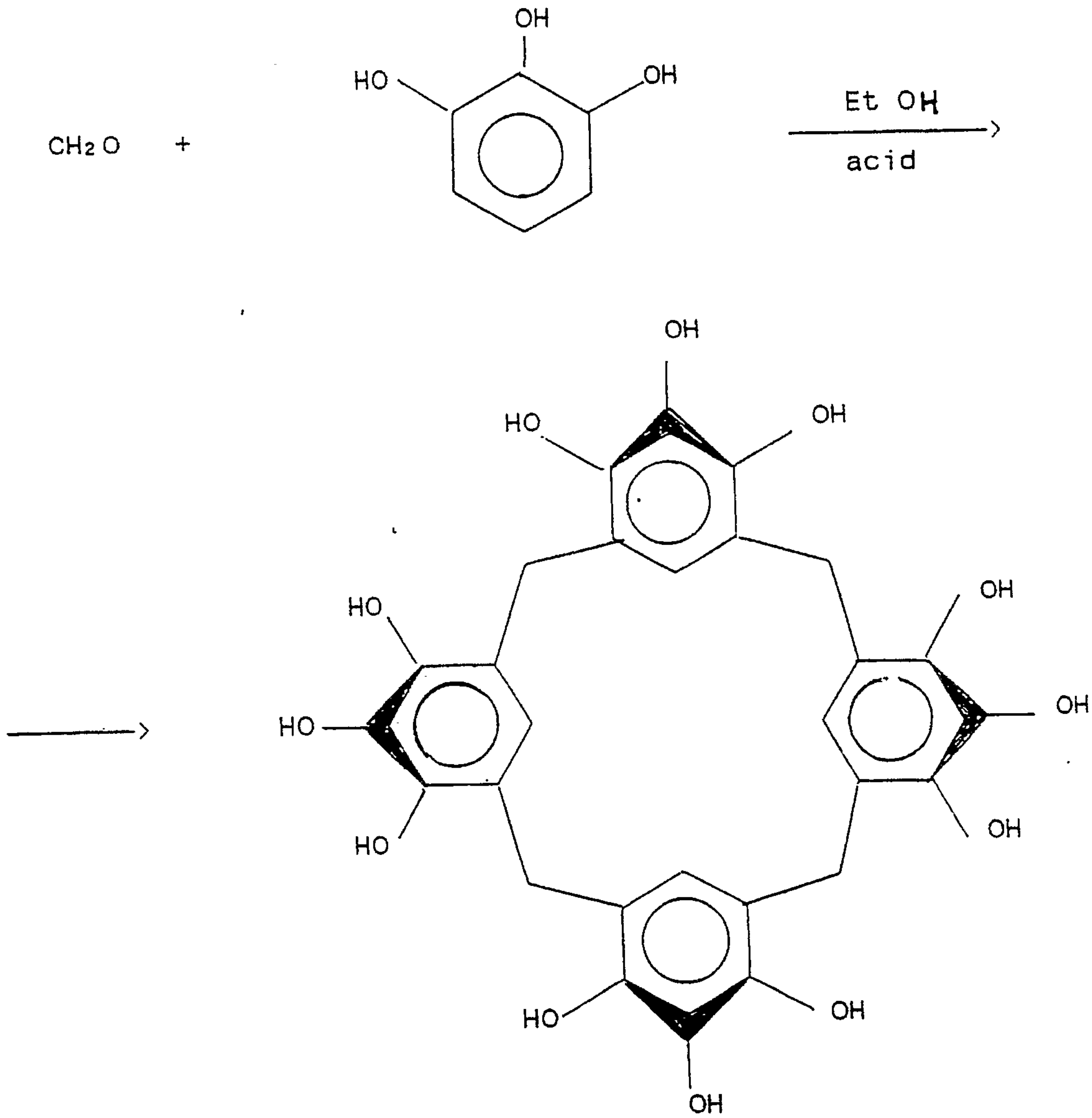


The macrocyclic tetramers according to the present invention display, owing to their same geometrical shape, an electric dipole moment oriented in the same direction as the $C_4$ symmetry axis of the molecules. The molecular dipole moments add up in the pyramidal mesophase, owing to the columnar arrangement of the molecules stacked on each other. As a result, the columns are polar, their polar axis coinciding with the axis of symmetry of the column.

If in all of the columns the direction of polarization is the same, such an organization at the microscopic level produces, at the macroscopic level, a ferroelectric material. This result can be obtained, e.g., by orienting the molecules by means of an external electrical field.

The presence of a conformational process of inversion of the macrocycle endows this class of liquid crystals with inherent ferroelectric properties: in other words, they show a hysteresis effect due to the inversion of polarization by an electrical field (two different, isoenergetic orientations as shown in Figure 3).

Such an inversion of the 16-membered macrocycle is much faster than the inversion obtainable by means of the rotation of the molecules.

Thanks to the above features, the pyramidal, columnar liquid crystals formed by the macrocycles according to the present invention can respond to an applied electrical field within extremely short periods of times.

These properties render the compounds of general formula I suitable for a wide range of applications, such as, e.g., as components for memory devices, electrooptical displays and in the field of photonics as

components for non-linear optical devices.

More generally, they can find an application in all of those fields in which liquid crystals are used.

The following non-limitative examples are given in order to illustrate the present invention.

EXAMPLE 1

Preparation of 3,4,5,10,11,12,17,18,19,24,25,26-dodecahydroxy-[$l_4$]-metacyclophane.

10.00 g (7.93 x $10^{-2}$ M) of pyrogallol and 2.60 g of paraformaldehyde were dissolved, under an argon atmosphere, in 80 ml of a 10% solution of hydrogen chloride in ethanol. The solution was kept stirred at 60 °C for 24 hours, then was heated to 85 °C and maintained at this temperature for 6 hours. At the end the resulting solution was poured into 600 ml of ethyl ether.

The so obtained precipitate was washed with ethyl ether, filtered and dried under a nitrogen atmosphere. 15.82 g of crude product were obtained and treated with an excess of butyryl chloride at 120 °C for 8 hours. At the end, the excess chloride was distilled off under vacuum, under a residual pressure of 20 torr. The residue was dissolved in methylene chloride and the resulting solution was first extracted with 2N NaOH, then washed with water until neutral, and finally thoroughly dried over anhydrous sodium sulfate. The resulting suspension was filtered, the solvent was evaporated to dryness and the residual crude reaction product was crystallized twice from acetonitrile/ethanol. 14.6 g of pure product were obtained (yield 13%).

Mass ($DCI^+$) : $MH^+$ = 1,393

$^1$H-N.M.R. ($CDCl_3$): δ 0.95 (M, 36H, $CH_3$); 1.65 (m, 24H, CO-$CH_2$-$\underline{CH}_2$); 2.40 (m, 24H, CO-$CH_2$); 3.61 (s, 8H, Ar-$CH_2$-Ar); 6.56 (s, 4H, Ar-H).

| * Elemental analysis for $C_{76}H_{96}O_{24}$. | |
|---|---|
| Theoretical:<br>Found : | C = 65.60%; H = 6.94%.<br>C = 65.48%; H = 7.34%. |

The pure macrocycle was obtained by saponification of the so obtained dodecabutyryl derivative according to the method described in J. Org. Chem. 1980, 45 , 4498-4500.

Mass ($DCI^+$): $MH^+$ = 553.

$^1$H-N.M.R. ($D_6$-acetone): δ 3.71 (s, 8H, Ar-$CH_2$-Ar); 6.60 (bs, 12H, OH); 6.80 (s, 4H, Ar-H).

EXAMPLE 2

Preparation of 3,4,5,10,11,12,17,18,19,24,25,26-dodecanonanoyloxy-[$l_4$]-metacyclophane.

0.554 g (1 mM) of the tetramer of Example 1 and 10.1 ml (30 mM) of nonanoyl chloride were heated with stirring for 12 hours at 140 °C. When the reaction was complete, the excess chloride was distilled off under a vacuum of $10^{-2}$ torr. The residue was dissolved in methylene chloride and the resulting solution was first extracted with 2N NaOH, then washed with water until neutral, and finally thoroughly dried over sodium sulfate. The solvent was evaporated to dryness and the residual crude reaction product was crystallized twice from ethanol and then purified on a silica gel column with methylene chloride as eluent. 1.72 g of pure product were obtained (yield 58%).

Mass ($DCI^+$): $MH^+$ = 2,234 (1 $^{13}$C)

$^1$H-N.M.R. ($CDCl_3$): δ 0.89 [t, 36H, $(CH_2)_n$-$\underline{CH}_3$]; 1.29 [bs, 120H, $(CH_2)_5$]; 1.61 (bm, 24H, CO-$CH_2$-$\underline{CH}_2$); 2.42 (t, 24H, CO-$CH_2$); 3.62 (s, 8H, Ar-$CH_2$-$\overline{Ar}$); 6.54 (s, 4H, Ar-H).

| * Elemental analysis for $C_{136}H_{216}O_{24}$. | |
|---|---|
| Theoretical : | C = 73.08%; H = 9.74%. |
| Found : | C = 73.10%; H = 10.03%. |

EXAMPLES 3-6

By operating in the. same way as described in Example 2, using decanoyl chloride, undecanoyl chloride, dodecanoyl chloride and tetradecanoyl chloride instead of nonanoylchloride, the following compounds were obtained:

3,4,5,10,11,12,17,18,19,24,25,26-dodecadecanoyloxy-[$l_4$]-metacyclophane: yield 46% (after being crystallized twice from ethanol).

Mass ($DCI^+$): $MH^+$ = 2,410 (1 $^{13}C$)

$^1$H-N.M.R. ($CDCl_3$): $\delta$ 0.87 [t, 36H, $(CH_2)_n$-$\underline{CH}_3$]; 1.30 [bs, 144H, $(CH_2)_6$]; 1.61 (bm, 24H, CO-$CH_2$-$\underline{CH}_2$); 2.40 (t, 24H, CO-$CH_2$); 3.60 (s, 8H, Ar-$CH_2$-$\overline{Ar}$); 6.54 (s, 4H, Ar-H).

| * Elemental analysis for $C_{148}H_{240}O_{24}$. | |
|---|---|
| Theoretical : | C = 73.96%; H = 10.06%. |
| Found : | C = 73.89%; H = 10.67%. |

3,4,5,10,11,12,17,18,19,24,25,26-dodecaundecanoyloxy-[$l_4$]-metacyclophane: yield 70% (after crystallization from ethanol).

Mass ($DCI^-$): $M^-$ = 2,269 (1 $^{13}C$)

$^1$H-N.M.R. ($CDCl_3$): $\delta$ 0.87 [t, 36H, $(CH_2)_n$-$\underline{CH}_3$]; 1.25 [bs, 168H, $(CH_2)_7$]; 1.60 (bm, 24H, CO-$CH_2$-$\underline{CH}_2$); 2.40 (t, 24H, CO-$CH_2$); 3.60 (s, 8H, Ar-$CH_2$-$\overline{Ar}$); 6.54 (s, 4H, Ar-H).

| * Elemental analysis for $C_{160}H_{264}O_{24}$. | |
|---|---|
| Theoretical : | C = 74.72%; H = 10.5%. |
| Found : | C = 74.70%; H = 10.45%. |

3,4,5,10,11,12,17,18,19,24,25,26-dodecadodecanoyloxy-[$l_4$]-metacyclophane: yield 64% (after crystallization from ethanol, followed by a purification on a silica gel column with 8/2 methylene chloride/hexane as eluent).

Mass ($DCI^-$): $M^-$ = 2,737 (1 $^{13}C$)

$^1$H-N.M.R. ($CDCl_3$): $\delta$ 0.87 [t, 36H, $(CH_2)_n$-$\underline{CH}_3$]; 1.25 [bs, 192H, $(CH_2)_8$]; 1.56 (bm, 24H, CO-$CH_2$-$\underline{CH}_2$); 2.40 (t, 24H, CO-$CH_2$); 3.60 (s, 8H, Ar-$CH_2$-$\overline{Ar}$); 6.54 (s, 4H, Ar-H).

| * Elemental analysis for $C_{172}H_{288}O_{24}$. | |
|---|---|
| Theoretical : | C = 75.39%; H = 10.59%. |
| Found : | C = 75.44%; H = 10.44%. |

3,4,5,10,11,12,17,18,19,24,25,26-dodecatetradecanoyloxy-[$l_4$]-metacyclophane: yield 53% (after being crystallized twice from ethanol, followed by a purification on a silica gel column with chloroform as eluent).

Mass ($DCI^-$): $M^-$ = 3,074 (2 $^{13}C$)

$^1$H-N.M.R. ($CDCl_3$): $\delta$ 0.88 [t, 36H, $(CH_2)_n$-$\underline{CH}_3$]; 1.28 [bs, 240H, $(CH_2)_{10}$]; 1.61 (bm, 24H, CO-$CH_2$-$\underline{CH}_2$); 2.42 (t, 24H, CO-$CH_2$); 3.59 (s, 8H, Ar-$CH_2$-$\overline{Ar}$); 6.53 (s, 4H, Ar-H).

| * Elemental analysis for $C_{196}H_{336}O_{24}$. | |
|---|---|
| Theoretical :<br>Found : | C = 76.51%; H = 10.01%.<br>C = 76.27%; H = 10.20%. |

EXAMPLES 7-8 (Comparative Examples)

By operating in the same way as described in Example 2, but using octanoyl chloride and palmitoyl chloride instead of nonanoyl chloride, two products not generating liquid crystal phases were obtained, i.e.: 3,4,5,10,11,12,17,18,19,24,25,26-dodecaoctanoyloxy-$[l_4]$-metacyclophane: yield 60% (after threefold crystallization from ethanol, followed by purification on a silica gel column with methylene chloride as eluent).
Mass (DCI$^-$): $MH^+$ = 2,065 (1 $^{13}C$)
$^1$H-N.M.R. ($CDCl_3$): δ 0.88 [t, 36H, $(CH_2)_n$-$CH_3$]; 1.29 [bs, 96H, $(CH_2)_4$]; 1.61 (bm, 24H, CO-$CH_2$-$CH_2$); 2.43 (t, 24H, CO-$CH_2$); 3.61 (s, 8H, Ar-$CH_2$-Ar); 6.53 (s, 4H, Ar-H).

| * Elemental analysis for $C_{124}H_{192}O_{24}$. | |
|---|---|
| Theoretical :<br>Found : | C = 72.06%; H = 9.36%.<br>C = 72.01%; H = 19.60%. |

3,4,5,10,11,12,17,18,19,24,25,26-dodecahexadecanoyloxy-$[l_4]$-metacyclophane: yield 50% (after purification on a silica gel column with 8/2 pentane/ethylether as eluent).
Mass (DCI$^-$): $M^-$ = 3,410 (2 $^{13}C$)
$^1$H-N.M.R. ($CDCl_3$): δ 0.88 [t, 36H, $(CH_2)_n$-$CH_3$]; 1.28 [bs, 288H, $(CH_2)_{12}$]; 1.59 (bm, 24H, CO-$CH_2$-$CH_2$); 2.42 (t, 24H, CO-$CH_2$); 3.58 (s, 8H, Ar-$CH_2$-Ar); 6.53 (s, 4H, Ar-H).

| * Elemental analysis for $C_{220}H_{384}O_{24}$. | |
|---|---|
| Theoretical :<br>Found : | C = 77.42%; H = 11.34%.<br>C = 77.41%; H = 11.56%. |

In Table 1 the transition temperatures and the enthalpy values of the products of examples 2 to 8 are reported.

TABLE 1

| Transition temperatures (°C), enthalpies (kJ/mol - in brackets) of dodeca-substituted tetramers, obtained by cooling (10°C/minute) from the isotropic phase and wherein: | | | | | | |
|---|---|---|---|---|---|---|
| $R' = CO\text{-}C_nH_{2n+1}$ | | | | | | |
| n | Yields | I | | C | | K |
| 7 | 60% | * | | | 74 (65.5) | |
| 8 | 58% | * | 63.2 (15.6) | * | 35 (69.0) | * |
| 9 | 46% | * | 65 (16.8) | * | 33 (103.2) | * |
| 10 | 70% | * | 52.4 (11.3) | * | 31 (70.5) | * |
| 11 | 64% | * | 54.2 (11.8) | * | 38 (102.2) | * |
| 13 | 53% | * | (#) | * | 46 (188.2) | * |
| 15 | 50% | * | | | 63.5 (262.0) | * |

I = isotropic phase
C = columnar liquid crystal phase of pyramidal type
K = crystal phase

* = observed phase
(#) mesophase observed under the optical microscope with polarized light

**Claims**

1. Thermotropic, dodeca-substituted, macrocyclic tetramers of general formula I:

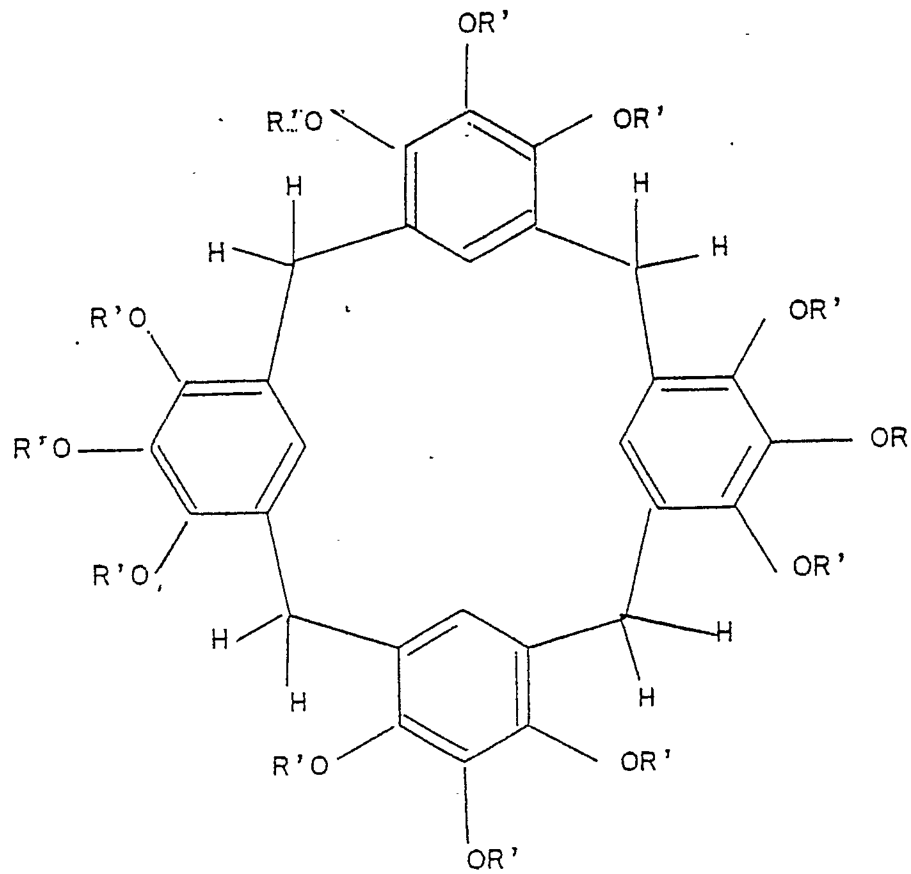

(I)

wherein the groups R', the same or different from one another, represent a radical of general formula II:
-COR (II)
wherein R is a linear alkyl group containing at least 8 carbon atoms.

2. Tetramers according to claim 1, in which R is a linear alkyl group containing from 8 to 13 carbon atoms.

3. Tetramers according to any one of claims 1 and 2, in which the groups R' are identical.

4. Tetramers according to any one of the preceding claims which have clearing points of from about 50 to about 100 °C, temperatures of formation of the mesophase, starting from the isotropic molten phase, of from about 50 to about 70 °C and crystallization temperatures of from about 30 to about 50 °C.

5. Tetramers according to any one of the preceding claims, wherein the mesophase is of the pyramidal columnar type.

6. Use of the tetramers of any one of the preceding claims as components for memory devices, electrooptical displays and in the field of photonics as components for non-linear optical devices.

7. Process for preparing the tetramers according to any one of claims 1 to 5, comprising reacting a tetramer of general formula III:

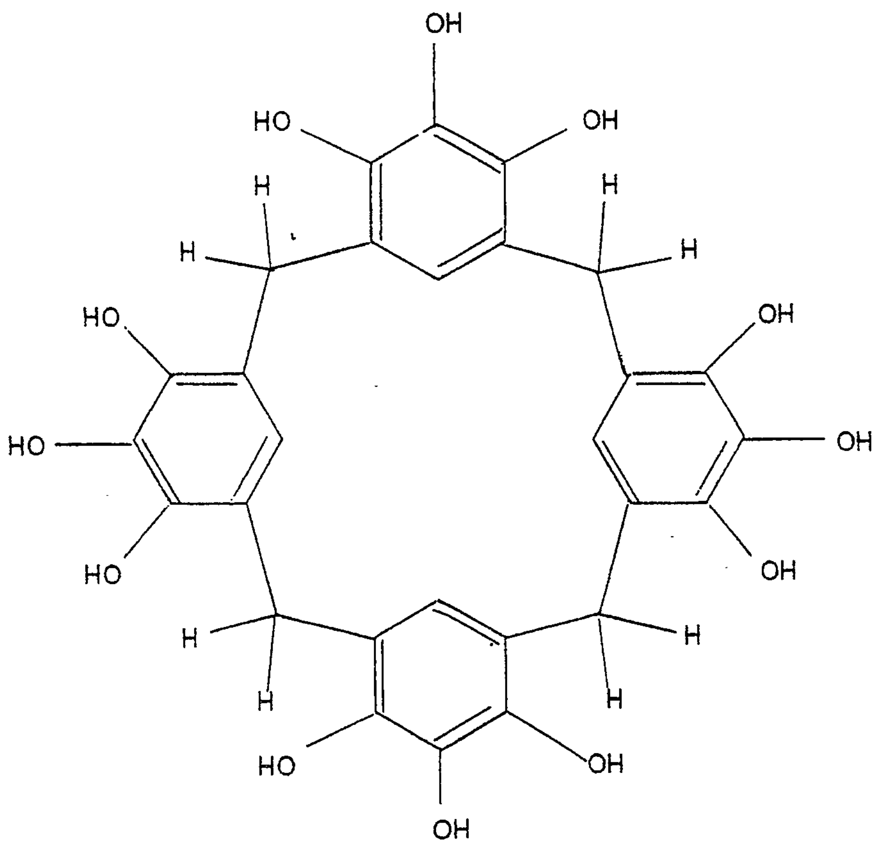

(III)

with one or more acyl halides of formula R'-X in which X represents halogen, particularly chlorine, and R' has the meaning given in claim 1.

8. Process according to claim 7, wherein the reaction is carried out at a temperature of from about 50 to about 200° C.

9. Macrocyclic tetramer of general formula III:

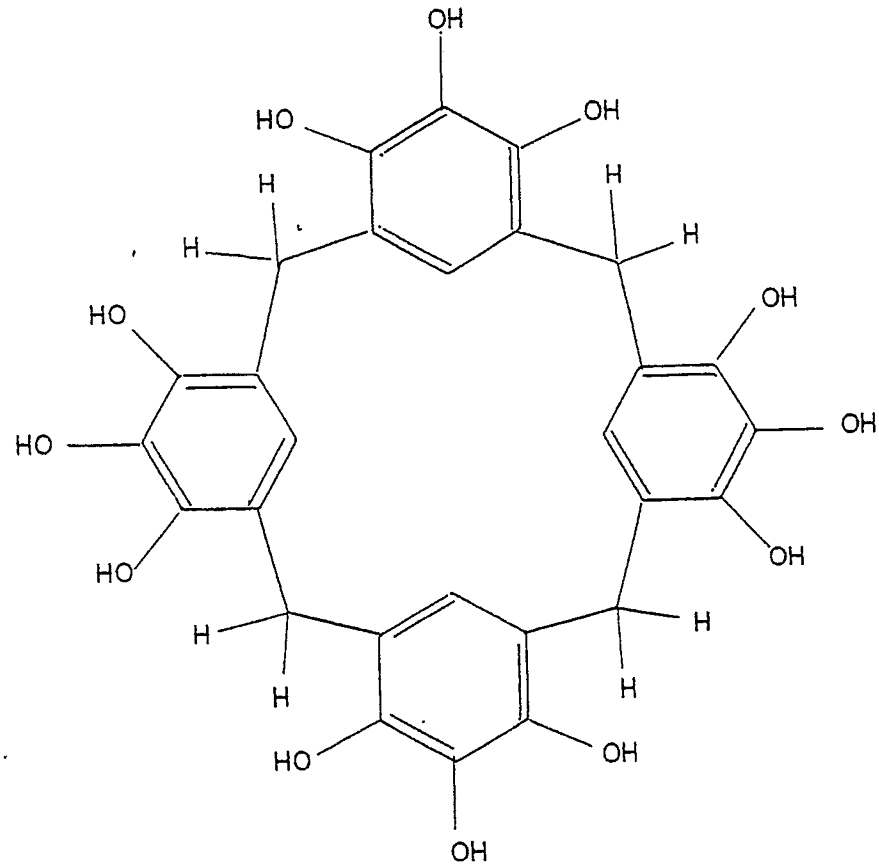

(III)

10. Process for preparing the macrocyclic tetramer of claim 9, comprising reacting at about 60 °C, pyrogallol and paraformaldehyde in ethanol under anhydrous conditions and in the presence of an acidic catalyst.

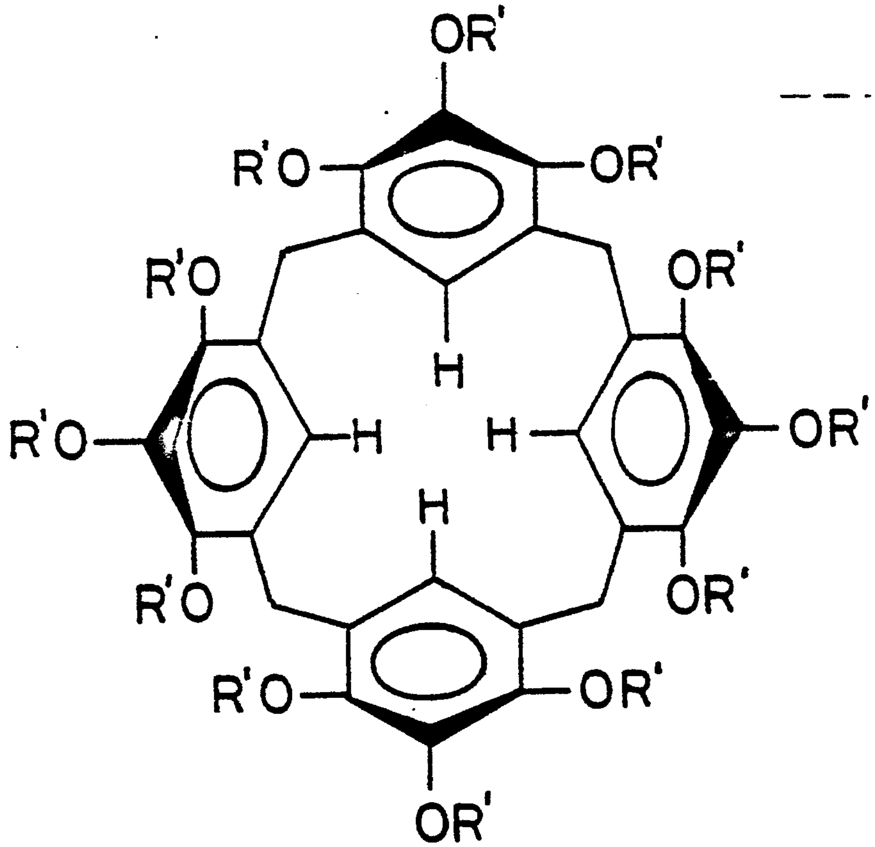

Fig.1

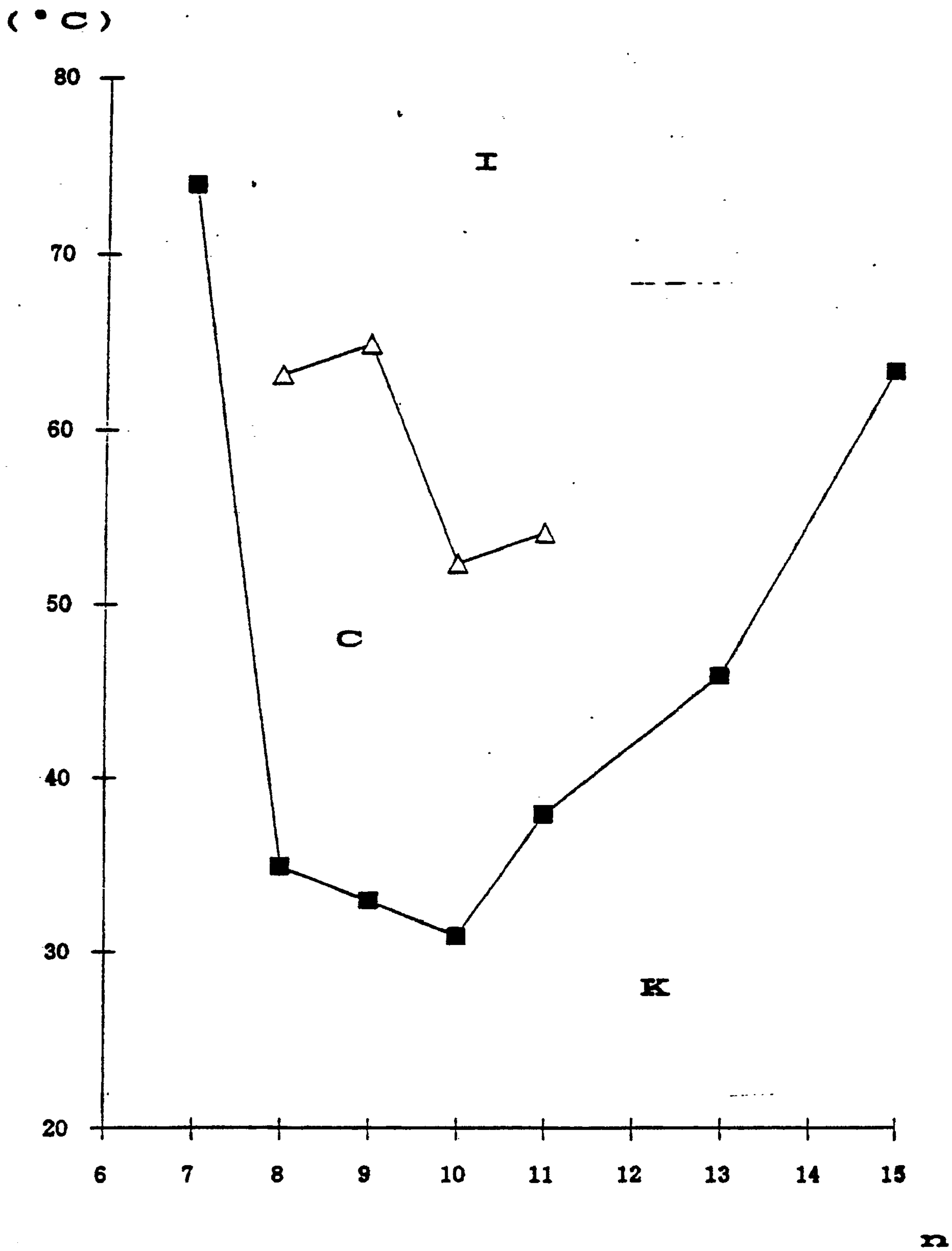
(°C)
80
70
60
50
40
30
20
6
7
8
9
10
11
12
13
14
15
n
I
C
K

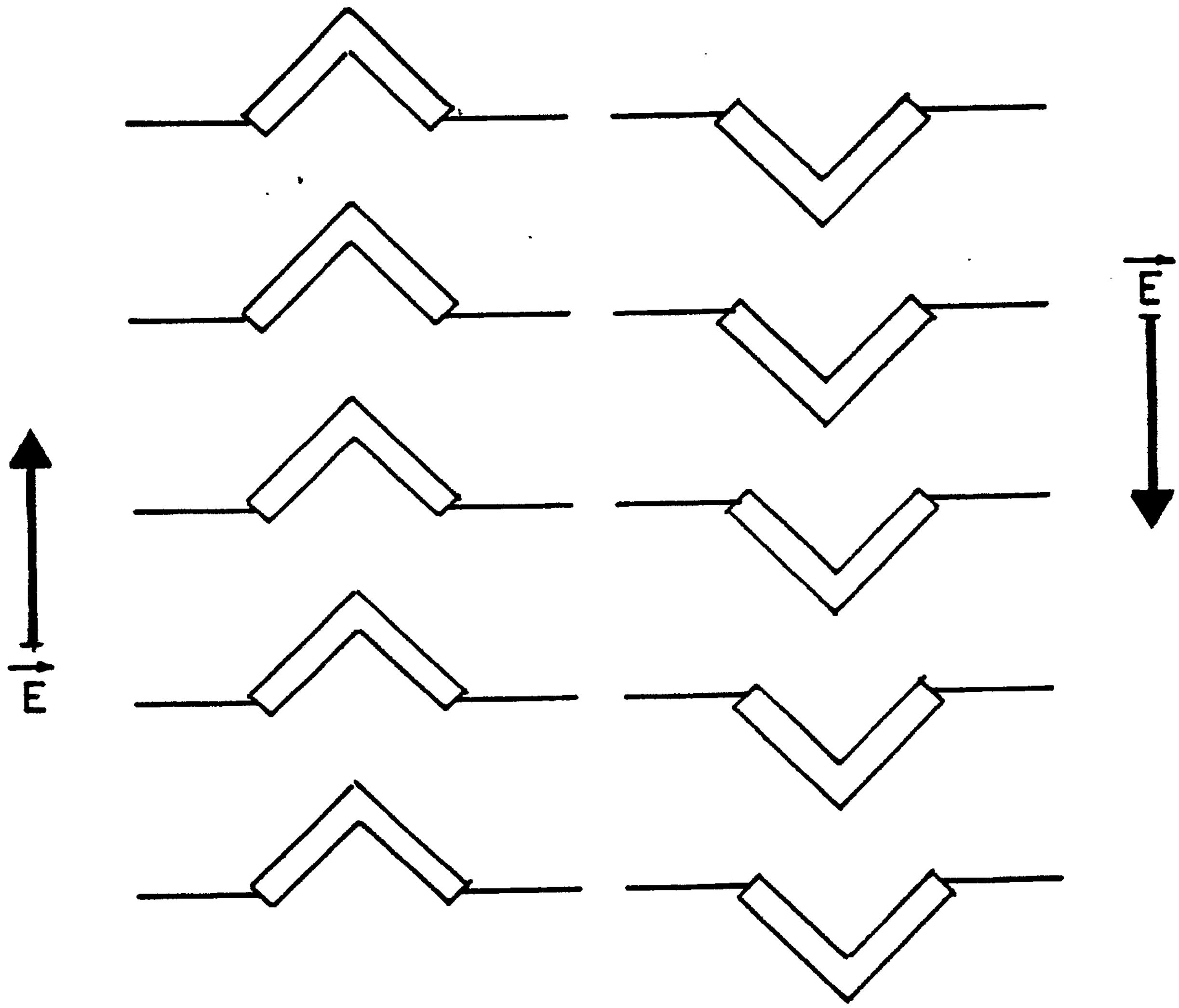


Fig.3